## Europäisches Patentamt
## European Patent Office
## Office européen des brevets

(19)

(11) Numéro de publication : **0 034 083**
**B1**

(12) # FASCICULE DE BREVET EUROPÉEN

(45) Date de publication du fascicule du brevet :
13.07.83

(51) Int. Cl.³ : **A 23 J   3/02**, A 61 K 37/18

(21) Numéro de dépôt : **81400117.8**

(22) Date de dépôt : **27.01.81**

(54) Procédé de traitement d'une matière à base de caséine contenant des phosphocaséinates de cations monovalents ou leurs dérivés, produits obtenus et applications.

(30) Priorité : **01.02.80 FR 8002281**

(43) Date de publication de la demande :
**19.08.81 Bulletin 81/33**

(45) Mention de la délivrance du brevet :
**13.07.83 Bulletin 83/28**

(84) Etats contractants désignés :
**AT BE CH DE FR GB IT LI LU NL SE**

(56) Documents cités :
EP A 0 022 019
FR A 2 292 435
FR A 2 399 213

CHEMICAL ABSTRACTS, vol. 87, no. 19, 7 novembre 1977, page 265, abrégé 148285p Columbus, Ohio, US D. W. WEST : « A simple method for the isolation of a phosphopeptide from bovine alpha s1-casein »

CHEMICAL ABSTRACTS, vol. 91, no. 21, 17 novembre 1979, page 523 abrégé 173597g Columbus, Ohio, US J. P. ROOZEN et al. : « Enzymic protein hydrolysis in a membrane reactor related to taste properties »

(73) Titulaire : **INSTITUT NATIONAL DE LA RECHERCHE AGRONOMIQUE (INRA)**
**149, rue de Grenelle**
**F-75341 Paris Cedex 07 (FR)**

(72) Inventeur : **Brule, Gérard**
**5, Cours de Lisbonne**
**F-35100 Rennes (FR)**
Inventeur : **Roger, Loic**
**27, rue de Brest**
**F-35000 Rennes (FR)**
Inventeur : **Fauquant, Jacques**
**Le Ricandais Pleumeleuc**
**F-35160 Montfort (FR)**
Inventeur : **Piot, Michel**
**10, rue du Bourbonnais**
**F-35000 Rennes (FR)**

(74) Mandataire : **Phélip, Bruno et al**
**c/o Cabinet Harlé & Phélip 21, rue de La Rochefoucauld**
**F-75009 Paris (FR)**

# Procédé de traitement d'une matière à base de caséine contenant des phosphocaséinates de cations monovalents ou leurs dérivés, produits obtenus et applications

La présente invention concerne le domaine du traitement des matières à base de caséine. Elle a plus particulièrement pour objet un procédé dans lequel on traite des phosphocaséinates de cations monovalents ou leurs dérivés. L'invention est également relative aux produits obtenus par un tel procédé, notamment des fractions enrichies en phosphopeptides et en peptides non phosphorylés, respectivement. L'invention concerne également les applications des produits ainsi obtenus, en particulier comme produits alimentaires devant répondre à des besoins spécifiques de la nutrition.

On sait que les caséines des matières premières laitières, et tout particulièrement du lait, contiennent des phosphosérines qui confèrent aux peptides dans lesquels elles se trouvent des propriétés physico-chimiques, technologiques et physiologiques intéressantes. On trouvera notamment des indications sur les protéines du lait, dans l'ouvrage de Mc KENZIE H. A. (1971) intitulé « Milk proteins » vol. 1 et 2 Academic Press New York.

L'ultrafiltration sur membranes, étant donné les progrès réalisés tant dans les appareillages que dans la compréhension des phénomènes observés, s'est largement répandue dans l'industrie laitière, pour le traitement du lait (voir par exemple Maubois J. L., Mocquot G. (1971) — Préparation de fromages à partir de pré-fromages liquides obtenus par ultrafiltration du lait — Revue « LE LAIT » fascicule *51* 508, 495-533). Lors du passage du lait sur la membrane d'ultrafiltration, l'eau, les sels minéraux solubles, le lactose, les composés azotés de faible poids moléculaire (peptides, acides aminés libres) et les vitamines hydrosolubles, traversent la membrane sous forme d'ultrafiltrat ou perméat, alors que les protéines et les constituants associés (calcium, phosphore), les globules gras et les éléments lipophiles sont retenus et se concentrent au fur et à mesure de l'élimination de la phase aqueuse : ils constituent le rétentat ou concentré protéique. L'obtention de concentrés protéiques de pureté élevée nécessite la mise en œuvre d'une ultrafiltration ainsi que d'une diafiltration. Dans la diafiltration, on effectue une addition d'eau ou de solution aqueuse contenant des sels, en continu ou en discontinu, dans le rétentat d'ultrafiltration. On élimine simultanément ou successivement une quantité équivalente de perméat. Une telle opération a pour conséquence d'appauvrir le rétentat en éléments filtrables. L'avantage de la technique d'ultrafiltration sur membranes est de conserver les protéines du lait à leur état natif.

Le procédé de l'invention tire profit de l'ultrafiltration sur membranes pour opérer un fractionnement des constituants des matières premières à base de caséine, mais en combinant cette ultrafiltration à une opération d'hydrolyse enzymatique.

On connaît un certain nombre de procédés pour l'hydrolyse des protéines, par exemple des protéines de lait. L'hydrolyse acide conduit effectivement à l'obtention de solutions d'acides aminés libres, mais elle en détruit certains. L'hydrolyse alcaline préserve le tryptophane mais entraîne une insolubilisation qui diminue fortement la valeur nutritionnelle des concentrés protéiques initiaux.

La protéolyse enzymatique est connue et pratiquée depuis fort longtemps à des fins analytiques ou à des fins alimentaires, le but principal étant de solubiliser les protéines. La littérature fait largement état de nombreuses utilisations alimentaires d'hydrolysats de protéines de soja [voir ARAI S., NOGUCHI M., KUROSAWA S., KATO H., et FUJIMAKI M. (1970) Applying proteolytic enzymes on soybean, 6-deodorization effect], des protéines de poissons : voir HEVIA P., WHITAKER J. R. et OLCOTT H. S. (1976) — Solubilization of a fish protein concentrate with proteolytic enzymes. J. Agric. Food Chem. Vol. 24 (2) 383-385, ou de colza, par action de protéase animale, microbienne ou végétale.

L'application de ces techniques aux protéines laitières au niveau industriel reste cependant très limitée.

La protéolyse enzymatique ne présente pas les inconvénients des méthodes chimiques. Les conditions d'hydrolyse sont modérées et préservent ainsi la qualité nutritionnelle des produits.

En général, l'hydrolyse conduit à des peptides ayant un goût amer prononcé. Ce caractère limite l'utilisation de ces hydrolysats en alimentation humaine. L'intensité de l'amertume d'un hydrolysat dépend principalement de la nature du substrat protéique et de la spécificité des enzymes. Pour éliminer l'amertume, on a suggéré de faire agir des exopeptidases. Voir par exemple ARAI S., YAMASHITA M., KATO H., FUJIMAKI M., (1970) Agric. Biol. Chem., 34, 729, ainsi que CLEGG K. M., SMITH G. et WALKER A. L., (1974) Production of an enzymatic hydrolysate of casein on a kilogram scale. J. Food Technol. *9*, 425-431. On a également proposé de modifier les peptides par addition d'acides glutamiques avant réaction plastéique. Il est également possible de recourir à une élimination des acides aminés hydrophobes.

Cependant, toutes ces techniques connues ne sont pas satisfaisantes et ne peuvent pas répondre aux besoins de l'invention. En effet, une solubilisation poussée, causée par la mise en œuvre d'exopeptidase, augmente le taux d'acides aminés libres et plus spécialement l'arginine, la lysine, la tyrosine, la valine, la phénylalanine, la méthionine et la leucine, ce qui aurait pour effet direct d'encombrer les systèmes de transport, au niveau de la barrière intestinale, des acides aminés libres, et donc d'entraîner une moindre efficacité nutritionnelle des hydrolysats. Par ailleurs, la qualité intrinsèque des hydrolysats est modifiée car l'équilibre en acides aminés est lui-même changé, ce qui nécessite une supplémentation en acides aminés libres.

Sur le plan technologique, l'hydrolyse enzymatique fait le plus souvent appel à un système de réacteur discontinu. L'enzyme est ajoutée à la solution protéique à traiter. Après un temps de séjour plus ou moins long, dans des conditions favorables à l'activité enzymatique et à l'attaque du substrat, le pH est modifié et un traitement thermique léger inactive l'enzyme. Une centrifugation peut être faite pour éliminer la fraction insoluble non digérée. Mais, selon cette technique de réaction d'hydrolyse enzymatique discontinue, il est difficile d'utiliser un rapport enzyme/substrat élevé. Or, on connaît, voir ROBINS R. C. (1978) — Effect of ratio of enzymes to substrate on amino acid patterns released from proteins in vitro. Internat. J. Vit. Nutr. Res., 48, 44-52, l'influence décisive du rapport enzyme/substrat sur la nature des acides aminés libres et les peptides libérés lors de la protéolyse. Avec un procédé discontinu, il faut détruire les enzymes en fin d'hydrolyse lorsque celles-ci sont en excès ce qui serait indispensable avec les rapports élevés précités.

On a également proposé d'utiliser des réacteurs à enzymes fixées. Cependant, ceux-ci présentent de notables inconvénients sur le plan pratique. En effet, les conditions optimales d'activités des enzymes, en particulier les conditions de pH, se déplacent, de sorte que le fonctionnement du réacteur ne donne pas satisfaction en permanence. On constate également des problèmes bactériologiques, le colmatage des lits de fixation ainsi que l'adsorption des protéines sur le support. En outre, la réaction enzymatique a tendance à s'inhiber au cours du temps en raison de la formation de complexe enzyme-fragment protéique. L'inhibition peut être également due à la nature du support. Il est par ailleurs très difficile d'utiliser des systèmes multi-enzymes en raison des phénomènes de compétition des enzymes vis-à-vis du support et de la stabilité différente des enzymes au cours du temps.

L'invention a mis à profit les moyens déjà connus dans certaines autres applications et qui consistent à faire appel à des réacteurs enzymatiques à membranes. On peut se référer par exemple à l'article de CHEFTEL C. (1972) — Solubilisation enzymatique continue du concentré protéique de poisson. Essai de recyclage des enzymes. Ann. Technol. Agric. 21, (3) 423-433, qui décrit un réacteur à membranes, appliqué à la protéolyse de concentrés protéiques de poisson. La membrane d'ultrafiltration permet de retenir l'enzyme en solution dans le réacteur ainsi que le substrat protéique. Seuls les produits de l'hydrolyse, les peptides, sont éliminés au fur et à mesure de leur formation. Cependant, dans la pratique, la mise en œuvre d'un tel réacteur n'est pas aisée comme le souligne CHEFTEL. Le substrat doit être complètement solubilisable par l'enzyme et la solution protéique doit présenter une qualité bactériologique irréprochable.

A titre de documents illustrant l'état de la technique, on peut encore citer les références ci-après :

le Brevet Français 77 24069 (publication 2.399.213) décrit le traitement d'un hydrolysat par ultrafiltration puis par électrodialyse. Ce document matérialise le fait qu'il était connu d'ultrafiltrer un hydrolysat protéique. Le procédé décrit dans ce brevet permet la production d'une solution pure d'acides aminés naturels ;

le Brevet Français 74 39311 (publication 2.292.435) concerne l'obtention de phosphocaséinate de calcium à partir d'un rétentat d'ultrafiltration du lait. L'enseignement de ce brevet a donc pour objet la production de phosphocaséinate de calcium. Il ne concerne pas le traitement des phosphocaséinates de cations monovalents ou de leurs dérivés ;

la référence CHEMICAL ABSTRACTS, volume 87, n° 19, 7 novembre 1977, page 265, abrégé 148, 285 p. COLUMBUS OHIO (US) & J. Dairy Research, volume 44, n° 2 (1977) pages 373-376 D. W. WEST « A simple method for the isolation of a phosphopeptide from bovine α sI-casein », décrit l'obtention d'un phosphopeptide à partir de caséinate. Le procédé implique une hydrolyse enzymatique par la trypsine, et des fractionnements par filtration sur gel et chromatographie, mais il débute par une réaction avec CNBr conduisant à des produits tout à fait spécifiques ;

la référence CHEMICAL ABSTRACTS, volume 91, n° 21, 19 novembre 1979, page 523, abrégé 173, 597 g COLUMBUS OHIO (US) & Enzyme Microb. Technol. vol. 1, n° 2 (1979) pages 122-124, J. P. ROOZEN et al. « Enzymic protein hydrolysis in a membrane reactor related to taste properties » décrit l'hydrolyse en réacteur enzymatique, dans le but d'améliorer le goût des hydrolysats protéiques. Ce document matérialise donc le fait que le réacteur enzymatique est un appareil connu.

Selon l'invention, le procédé de traitement est appliqué à des solutions protéiques dépourvues d'ions bivalents, tels que le calcium et le magnésium. On utilise essentiellement, en effet, des phosphocaséinates de cations monovalents ou leurs dérivés.

Sous sa forme la plus générale, le procédé de l'invention est caractérisé en ce qu'on soumet des phosphocaséinates de cations monovalents ou leurs dérivés à une hydrolyse enzymatique en soi connue à l'aide d'au moins une enzyme protéolytique capable de reconstituer la digestion protéique qui prend place in vivo dans l'organisme humain, ladite hydrolyse enzymatique étant réalisée à un pH entre 7 et 9 et à une température de 37 à 45 °C, en ce qu'on soumet l'hydrolysat obtenu à au moins une ultrafiltration sur des membranes laissant passer dans le perméat tous les peptides de l'hydrolysat, en ce qu'on ajoute au perméat au moins un sel de cation bivalent pouvant former des agrégats avec la fraction phosphorylée desdits peptides, ce qui conduit à une solution contenant essentiellement des agrégats de phosphopeptides et des peptides non phosphorylés, et en ce qu'on sépare par au moins une ultrafiltration les peptides non phosphorylés et les phosphopeptides, lesquels ont une grosseur de particule supérieure, par mise en contact de la solution avec au moins une membrane capable de retenir les phosphopeptides.

Les matières premières à base de caséine susceptibles d'être traitées par le procédé de l'invention contiennent des phosphocaséinates de cations monovalents, tels que les phosphocaséinates de sodium,

3

de potassium ou d'ammonium. On peut également traiter une matière première contenant des dérivés desdits phosphocaséinates, notamment la paracaséine. Tous ces composés sont connus de l'homme de l'art et accessibles par des voies industrielles. Par exemple, pour préparer des caséinates monovalents tels que le caséinate de sodium, on prépare d'abord la caséine, par exemple en partant de lait, par précipitation au point isoélectrique. Après lavage de celle-ci, on additionne au précipité de caséine de l'hydroxyde de sodium, hydroxyde de potassium, hydroxyde d'ammonium ou autres composés basiques comportant des ions monovalents, et capables de résolubiliser la caséine. On obtient finalement une solution protéique contenant lesdits caséinates de cations monovalents, de préférence les caséinates de sodium ou de potassium. De telles substances peuvent servir directement de matière première pour le procédé de l'invention.

En variante, on peut utiliser des dérivés desdits caséinates, en particulier sous forme de paracaséine. A cet effet, on traite au préalable la solution de phosphocaséinate de cations monovalents en lui ajoutant de la présure, qui provoque une réaction d'hydrolyse. Le produit d'hydrolyse contient des paracaséinates, et les caséinomacropeptides (CMP). On effectue ensuite la précipitation de la paracaséine par tout moyen connu, de préférence par acidification à pH 4,6, avec un acide alimentaire quelconque, organique ou inorganique, par exemple l'acide chlorhydrique, l'acide phosphorique, l'acide sulfurique, l'acide acétique, l'acide lactique et autres acides analogues. Dans la pratique, on préfère l'acide chlorhydrique. On sépare alors la solution surnageante, qui contient les caséinomacropeptides, de la paracaséine précipitée. Cette dernière sert à son tour de matière première pour le procédé de l'invention. Dans cette variante, on obtient une solution contenant du caséinomacropeptide qui peut constituer un produit de valeur. Pour le purifier et le séparer, on peut neutraliser ladite solution par un composé basique, tel que l'hydroxyde de sodium. Le CMP peut être préparé sous forme concentrée par ultrafiltration de la solution, après addition de chlorure de calcium.

Dans une forme de réalisation préférée de la variante décrite ci-dessus, le caséinate de sodium en solution dans l'eau (3 %) a été hydrolysé par la présure (20 ml/100 l). La paracaséine a été ensuite précipitée par acidification (pH 4,6) avec HCl. La solution surnageante contenant le caséinomacropeptide a été ensuite neutralisée (pH 7,0) par l'hydroxyde de sodium, et concentrée par ultrafiltration après addition de 0,5 g de $CaCl_2$. A partir de 1 000 litres de solution de caséinate à 3 %, cette variante permet d'obtenir environ 30 à 40 litres de solution de caséinomacropeptide à 3 %.

Quelle que soit la matière première utilisée dans le procédé de l'invention, la première étape consiste en une hydrolyse enzymatique à l'aide d'au moins une enzyme protéolytique capable de reconstituer la digestion protéique qui prend place in vivo dans l'organisme humain. Ainsi qu'on l'a mentionné précédemment, une telle hydrolyse est avantageusement mise en œuvre dans un dispositif qui combine un équipement d'ultrafiltration à un réacteur enzymatique, permettant ainsi une réalisation continue.

Dans un tel mode de réalisation, l'hydrolyse enzymatique est réalisée en continu en introduisant la matière première à base de caséine dans une zone de réaction en vue d'une mise en contact intime avec l'enzyme, on soutire en continu le produit de réaction en l'amenant de la zone de réaction dans une zone d'ultrafiltration, de laquelle on soutire également en continu un perméat qui constitue l'hydrolysat peptidique.

Au cours de l'hydrolyse enzymatique, le pH doit être ajusté entre 7 et 9. A cet effet, on introduit, en continu ou en discontinu, dans la zone de réaction, un composé basique qui peut être l'hydroxyde ou le carbonate de sodium, l'hydroxyde ou le carbonate de potassium, l'hydroxyde d'ammonium ou un mélange de ces produits. Le choix d'un composé basique particulier dépendra de la destination du produit final.

A titre d'enzyme, on met en œuvre de préférence au moins une enzyme protéolytique capable de reconstituer la digestion protéique qui prend place in vivo dans l'organisme humain. On utilise donc avantageusement la pancréatine qui est un mélange complexe contenant de la trypsine, de la chymotrypsine et d'autres enzymes protéolytiques secondaires. Dans la pratique, on peut faire appel à un extrait pancréatique naturel disponible sur le marché et aisément accessible. Toutefois, si besoin est, on peut également mettre en œuvre des enzymes résultant d'un mélange synthétique, par exemple d'alphachymotrypsine et de trypsine. De préférence, on utilise un mélange synthétique dont la composition se rapproche de la pancréatine, avec par conséquent présence des enzymes secondaires contenues dans l'extrait pancréatique naturel. On a trouvé selon l'invention qu'au pH compris entre 7 et 9, et de préférence entre 7 et 8,5, par exemple à 8, la pancréatine et autres enzymes analogues répondant aux besoins de l'invention présentaient une stabilité maximale.

Il convient d'observer également des conditions assez strictes de température dans la zone d'hydrolyse enzymatique. On a constaté en effet que l'activité des enzymes était davantage influencée par la température que par le pH. Des essais ont montré notamment, selon l'invention, qu'avec la trypsine la température maximale au cours de l'hydrolyse enzymatique ne devait pas être supérieure à 54 °C, et, avec la chymotrypsine, cette température ne devait pas dépasser 45 °C. Dans la pratique, lorsque l'on utilisera la pancréatine, on adoptera un compromis tenant compte à la fois des conditions optimales de la protéolyse intestinale in vivo (température de l'ordre de 37 °C) et du fait que des températures plus élevées sont moins favorables au développement des germes et permettent d'obtenir des débits d'ultrafiltration supérieurs. En général, on choisit des températures de l'ordre de 37 à 40 °C, et de préférence encore voisines de 37 °C.

Il va sans dire que les paramètres de la réaction, c'est-à-dire le pH et la température d'hydrolyse enzymatique, sont liés. L'homme de l'art pourra donc choisir les conditions les plus favorables dans chaque cas d'espèce.

Pour réaliser une hydrolyse enzymatique optimale, il convient également de choisir avec soin la membrane d'ultrafiltration à utiliser en relation avec le réacteur. Les membranes utilisées sont de type quelconque, organique ou inorganique. Une organisation des membranes ayant donné de bons résultats est celle des modules à fibres creuses. A titre particulier, on peut utiliser les membranes de la société AMICON disponibles sur le marché sous la dénomination H 10P P 5 (seuil de coupure 5 000) et H 10 P 10 (seuil de coupure 10 000) ainsi que les membranes de la société ROMICON disponibles sur le marché sous la dénomination PM 2 (seuil de coupure 2 000) ou PM 50 (seuil de coupure 50 000). La seule condition à respecter est que la membrane, en fonctionnement, retienne efficacement l'enzyme, tout en présentant des performances satisfaisantes, en particulier sur le plan de la longévité.

Le procédé de l'invention peut être mis en œuvre en deux étapes séparées : une première étape consistant en l'hydrolyse enzymatique, et une deuxième étape consistant en l'ultrafiltration associée à ladite hydrolyse. Les équipements pour la mise en œuvre de chacune de ces opérations peuvent être séparés ou intégrés. Mais, en variante, le procédé peut également être exécuté en continu, les deux étapes ci-dessus étant réalisées dans un appareillage unique. Pendant les premiers instants de fonctionnement, par exemple pendant une heure environ, le perméat (liquide passant à travers la membrane) est recyclé dans la zone d'hydrolyse afin d'obtenir le taux d'hydrolyse désiré de la matière à base de caséine. Après l'hydrolyse, on introduit dans le réacteur la matière première à base de caséine à traiter à un débit identique à celui du perméat.

Ainsi, une forme préférée de réalisation de l'invention consiste à combiner et à mettre en œuvre en continu l'hydrolyse enzymatique et l'ultrafiltration sur membrane, ce qui permet de récupérer dans le perméat tous les peptides de l'hydrolysat. Les caractéristiques de la membrane d'ultrafiltration utilisée en combinaison avec l'hydrolyse enzymatique doivent être telles qu'elle laisse passer librement tous les peptides de l'hydrolysat. Des membranes ayant un pouvoir de coupure de 50 000 ou supérieur se sont avérées convenables.

Conformément à une caractéristique essentielle de l'invention, on ajoute ensuite au perméat au moins un sel de cations bivalents pouvant former des agrégats avec la fraction phosphorylée des peptides. On a constaté en effet que la complexation des cations bivalents facilite l'agglomération des phosphopeptides entre eux, ce qui permet de les séparer des peptides non phosphorylés. La séparation des phosphopeptides et des peptides non phosphorylés obtenus après hydrolyse enzymatique repose sur l'aptitude des phosphosérines à complexer les alcalinoterreux, en particulier les ions calcium et magnésium. Lorsque, comme c'est le cas du procédé de l'invention, l'hydrolyse s'effectue sur des solutions protéiques dépourvues de calcium et/ou de magnésium, il importe d'ajouter aux solutions peptidiques obtenues après hydrolyse les cations bivalents en cause, qui exercent la fonction de complexation.

Dans la pratique, on préfère utiliser, à titre de cations bivalents de complexation, des cations calcium, par exemple qui sont apportés par le chlorure de calcium. La quantité d'agent de complexation des peptides à base de cations bivalents qui, conformément à l'invention, doit être ajoutée à la solution peptidique, n'est pas critique. Dans la pratique, des quantités de chlorure de calcium de l'ordre de 0,5 % en poids par rapport à la solution de peptides ont été jugées convenables.

Il va sans dire que l'homme de l'art pourra choisir les composés bivalents et la quantité à mettre en œuvre, en tenant compte également des caractéristiques de l'étape consécutive de séparation entre les agrégats de phosphopeptides et les peptides non phosphorylés, ladite séparation étant, conformément à l'invention, effectuée par une ultrafiltration. En d'autres termes, il faudra notamment tenir compte du seuil de coupure de la membrane d'ultrafiltration, de manière que les agrégats phosphopeptidiques ne traversent pas la membrane.

Selon un mode de réalisation qui a donné de bons résultats, on associe à l'agent de complexation, un phosphate minéral, tel que le phosphate acide de sodium $PO_4HNa_2$. La présence d'un tel composé de phosphate peut favoriser la complexation et la formation de gros agrégats phosphopeptidiques. Dans certains cas cependant, en particulier si l'on souhaite obtenir à l'issue du procédé des fractions de phosphopeptides qui ne sont pas enrichies exagérément en phosphate minéral, on peut diminuer la quantité de phosphate ajoutée, et même y renoncer totalement, dès lors qu'on utilise à la fin du procédé une membrane capable de retenir les phosphopeptides, dont le seuil de coupure se situe de préférence entre 2 000 et 50 000, et avantageusement entre 2 000 et 10 000.

Ainsi qu'on l'a mentionné précédemment, chacune des ultrafiltrations ci-dessus peut être suivie d'une diafiltration au cours de laquelle on ajoute, en continu ou en discontinu, un liquide tel que l'eau ou une solution saline, en vue de purifier davantage les produits de l'ultrafiltration. Dans le procédé de l'invention, l'eau s'est avérée un moyen de diafiltration approprié.

Comme résultat de l'ultrafiltration et de la diafiltration qui suivent l'hydrolyse enzymatique, on obtient, d'une part, une solution peptidique qui est traitée complémentairement par le procédé de l'invention, et d'autre part une fraction (rétentat) constituée de résidus protéiques et d'enzymes résiduelles. Comme résultat de l'ultrafiltration et de la diafiltration réalisées à la fin du procédé de l'invention, on obtient d'une part, à titre de perméat, des peptides non phosphorylés et, d'autre part, à titre

5

0 034 083

de rétentat, des phosphopeptides.

Dans un mode de mise en œuvre préféré, la préparation des fractions peptidiques a été effectuée à partir d'une solution de caséinate de sodium à 6 %. L'hydrolyse en réacteur enzymatique a été réalisée avec de la pancréatine à raison de 4 g/l à pH 8 et à 37 °C. Le contenu du réacteur a été ensuite diafiltré à l'eau. La solution de peptides a été alors acidifiée à pH 6,2, l'agrégation étant réalisée par addition de $CaCl_2$ (0,5 %) et de $PO_4HNa_2$ (0,2 %). La solution de peptides a été ensuite ultrafiltrée et diafiltrée. En partant de 1 000 litres de caséinate de sodium à 6 %, on peut obtenir 900 litres de solution peptidique non phosphorylée à 45 g/l et 100 à 120 litres de solution de phosphopeptides à 80 g/l.

Les phosphopeptides ainsi obtenus à l'issue du procédé de l'invention constituent le produit de valeur le plus intéressant. Il présente en effet une teneur élevée en phosphosérines et une faible teneur en acides aminés aromatiques (phénylalanine, tyrosine, tryptophane).

La fraction obtenue, riche en phosphopeptides, peut donc se caractériser par sa composition particulière en acides aminés ainsi que par une teneur élevée en matière minérale (cendres) par rapport à l'azote total, étant donné que la fraction phosphopeptidique a complexé les sels ajoutés.

Le tableau I qui suit indique les principales caractéristiques des produits de l'invention, avec à la première colonne, celles du caséinate monovalent à titre de référence.

Tableau I

| | Caséinate de K ou Na | Peptides non phosphorylés | Phosphopeptides |
|---|---|---|---|
| Somme des acides aminés aromatiques (Tyr, Phe, Trp) | 12 % | > 12 % | < 4 % |
| Serines | 4,9 % | < 4 % | < 20 %<br>> 8 % |
| $\dfrac{Ca + Mg + P}{N_T{}^{1)}}$ | < 0,01 | < 0,02 | > 0,2 |
| Acides aminés libres | — | < 10 % | < 3 % |

$^{1)}$ $N_T$ = azote total × 6,38.

Les phosphopeptides obtenus grâce au procédé de l'invention peuvent recevoir de nombreuses applications dans le domaine alimentaire.

Les produits de l'invention sont utiles dans l'alimentation, en particulier dans l'alimentation humaine, et dans la nutrition thérapeutique. On sait en effet que, dans le lait humain, le phosphore dit organique, c'est-à-dire lié aux protéines et lié aux lipides, est relativement plus important que dans d'autres laits, notamment le lait de vache. Ainsi, le rapport :

$$\frac{\text{phosphore organique}}{\text{phosphore total}}$$

est d'environ 0,83 dans le lait humain contre 0,34 dans le lait de vache. Plus précisément, le rapport :

$$\frac{\text{phosphore organique lié à l'azote}}{\text{phosphore inorganique}}$$

est d'environ 0,70 dans le lait humain contre 0,36 dans le lait de vache. Les produits de l'invention trouvent donc application dans le domaine connu sous le nom de maternisation des laits.

Mais, d'une façon générale, il est reconnu que la qualité essentielle des protéines du lait de femme est d'assurer un anabolisme azoté remarquable associé à une charge osmotique rénale et une charge en ions $H^+$ particulièrement faibles.

Or, cette conjonction anabolisme azoté très élevé, charge osmotique rénale et charge en ions $H^+$ faibles est particulièrement recherchée en réanimation et en nutrition thérapeutique où coexistent très souvent des besoins d'anabolisme élevé et une insuffisance rénale fonctionnelle.

Les produits de l'invention peuvent permettre de répondre à ce besoin. Les produits de l'invention, pour certaines applications, ont une teneur insuffisante en certains acides aminés essentiels (phénylalanine, tyrosine, tryptophane, cystine). Ils peuvent alors avantageusement être associés à d'autres protéines ou peptides ou homologues alpha-céto-acides ou alpha (OH) acides d'acides aminés essentiels, pour que

6

soit rétabli un bon équilibre en acides aminés aboutissant à une valeur biologique optimale.

On notera aussi que de tels produits (phosphopeptides) ont une affinité élevée vis-à-vis des macroéléments (calcium, magnésium) et des oligo-éléments tels que, particulièrement, le fer, le zinc, le cuivre, le chrome, le nickel, le cobalt, le manganèse et le sélénium.

Les phosphopeptides selon l'invention peuvent être avantageusement transformés en sels desdits éléments par des moyens usuels. Ainsi, pour obtenir un tel sel organophosphoré, on peut utiliser, à titre de solution de diafiltration lors de la purification des phosphopeptides, une solution du sel contenant l'élément à introduire, par exemple une solution de chlorure de fer dans le cas du fer. Ces sels organophosphorés sont très solubles et peuvent avantageusement être utilisés pour véhiculer les éléments en cause.

Les produits de l'invention répondent aux besoins nutritionnels des malades souffrant d'insuffisance pancréatique, de maladies métaboliques, d'insuffisance ou de détresse nutritionnelle associée ou non à une insuffisance rénale fonctionnelle ou organique, en particulier lorsqu'ils sont associés à des peptides, des acides aminés essentiels ou des homologues d'acides aminés essentiels.

L'invention trouve donc une application directe dans des aliments diététiques ou des produits de nutrition thérapeutique parfaitement assimilables par l'organisme humain.

Quelle que soit la source de protéines, de peptides ou d'acides aminés utilisés, ces phosphopeptides permettent de réguler, de la manière la plus souhaitable, le taux de phosphore organique lié à l'azote dans la formule que l'on désire créer.

Ainsi qu'on l'a mentionné précédemment, les phosphopeptides selon l'invention ainsi que leurs dérivés, notamment les sels organophosphorés qu'ils forment avec des éléments minéraux, tels que le calcium ou le magnésium, et/ou avec des oligoéléments (Fe, Zn, Cu, Cr, Ni, Co, Mn, Se par exemple) trouvent une application intéressante en diététique.

L'invention a donc pour objet des compositions diététiques contenant une quantité efficace d'au moins un tel phosphopeptide ou dérivé de phosphopeptide en association avec un véhicule acceptable du point de vue nutritionnel. La quantité efficace peut varier dans de larges limites selon l'effet recherché. A titre indicatif, une quantité de 10 % en poids par rapport au total de la composition convient dans les cas habituels.

Les produits de l'invention peuvent également trouver une application en tant que tels, à titre de médicaments pour l'homme et l'animal.

Les médicaments en cause sont appropriés pour lutter contre toutes les affections impliquant une carence en phosphore organique et en certains éléments minéraux. On donnera ci-après, à titre illustratif et nullement limitatif, des exemples concrets de telles applications.

Les dérivés minéraux des phosphopeptides selon l'invention, consistant en leurs sels de calcium, constituent un supplément minéralo-protidique riche en phosphore organique et en calcium. Ils trouvent une application comme médicaments, par exemple dans les cas suivants :
— recalcification des os après fracture,
— traitement de l'ostéoporose,
— supplémentation calcique au cours du traitement du rachitisme.

Les dérivés des phosphopeptides selon l'invention consistant en leurs sels de magnésium constituent un supplément minéralo-protidique riche en phosphore organique et en magnésium. Ils trouvent une application comme médicaments pour remédier à toutes les formes de déficit magnésique, en particulier chez l'adulte, par exemple dans les cas suivants :
— besoins en Mg fortement accrus par le stress,
— mauvaise utilisation du Mg alimentaire chez les vieillards,
— augmentation des besoins en Mg chez la femme enceinte.

Des médicaments contenant des dérivés de phosphopeptides consistant en des sels mixtes de calcium et de magnésium sont utilisables de la même manière à titre de supplément minéralo-protidique.

Il va sans dire que des applications analogues peuvent être prévues pour des sels variés des phosphopeptides selon l'invention, bien que, dans la pratique, les sels de calcium et/ou de magnésium soient préférés.

On notera que les phosphopeptides, tels qu'ils sont obtenus par le procédé de l'invention, se trouvent sous forme de leurs sels de cations bivalents, en particulier de sels de calcium et/ou de magnésium. Si besoin est, ces sels peuvent fournir des phosphopeptides neutres par abaissement du pH du milieu, par exemple jusqu'à 4,6 environ. Mais, dans la pratique, cette opération n'est pas nécessaire car les sels de phosphopeptides conviennent parfaitement pour les utilisations. Ainsi, lorsque les phosphopeptides de l'invention se trouvent inclus dans des compositions diététiques ou pharmaceutiques, ils se trouvent sous forme de sels, par exemple de calcium et/ou de magnésium.

Les macroéléments (calcium et/ou magnésium, de préférence) peuvent être remplacés, au moins partiellement, par des oligoéléments.

Les dérivés de phosphopeptides selon l'invention qui contiennent des oligoéléments, trouvent une application correspondant à celle du ou des oligoéléments particuliers.

Les indications générales des médicaments contenant des dérivés de phosphopeptides et d'oligoéléments sont notamment les malabsorptions digestives induisant des carences en oligoéléments (Fe, Zn, Cu, Cr, Ni, Mn, Se). Ces malabsorptions digestives apparaissent en particulier lors d'iléites inflammatoires, dans le cas d'intestins réséqués, maladies cœliaques et intestins radiques. A titre d'exemples, les carences en zinc peuvent provoquer l'acrodermite entéropathique, des diarrhées, une susceptibilité accrue aux infections, l'hypogonadisme. Les carences en fer peuvent entraîner de l'anémie sidéropénique.

Les médicaments selon l'invention sont préférés pour le traitement des carences en zinc, cuivre, chrome et fer.

L'invention concerne aussi des compositions pharmaceutiques contenant un produit de l'invention en mélange avec les excipients usuels. Compte tenu de la forme physique du nouveau produit (poudre soluble en milieu aqueux), la forme de présentation ne soulève pas de difficulté. Les nouveaux produits peuvent être ingérés ou administrés en tant que tels, notamment par voie entérale, par exemple en mélange avec l'aliment habituel. Ils peuvent également être présentés sous forme de compositions avec des excipients usuels par exemple convenant à l'administration orale. Des compositions appropriées aux fins de l'invention peuvent ainsi être présentées sous forme de comprimés ou de gélules, avec des excipients connus tels que le talc, le stéarate de magnésium, la silice finement divisée, et tout autre véhicule analogue connu de l'homme de l'art.

A titre d'exemple, on a indiqué ci-après un cas particulier illustrant la préparation de compositions pharmaceutiques pour l'administration par voie orale. Des comprimés ont été préparés de la manière usuelle à partir de la formulation suivante :

| | |
|---|---|
| phosphopeptide (ou sel de phosphopeptide) selon l'invention | 200 mg |
| excipient QS pour un comprimé terminé à | 300 mg |

L'excipient utilisé peut être le talc, le stéarate de magnésium ou la silice disponible sur le marché sous la dénomination « aérosil ».

On a préparé de la même manière des gélules dosées à 100 mg de phosphopeptide (ou de sel) selon l'invention et contenant un excipient usuel QS pour une gélule terminée à 200 mg.

L'invention sera maintenant illustrée par la description ci-après et les exemples qui suivent.

La description est faite en référence aux dessins annexés sur lesquels :

Figure 1   représente schématiquement les diverses étapes du procédé de l'invention.

Figure 2   est un schéma d'un réacteur enzymatique utilisable dans le procédé de l'invention.

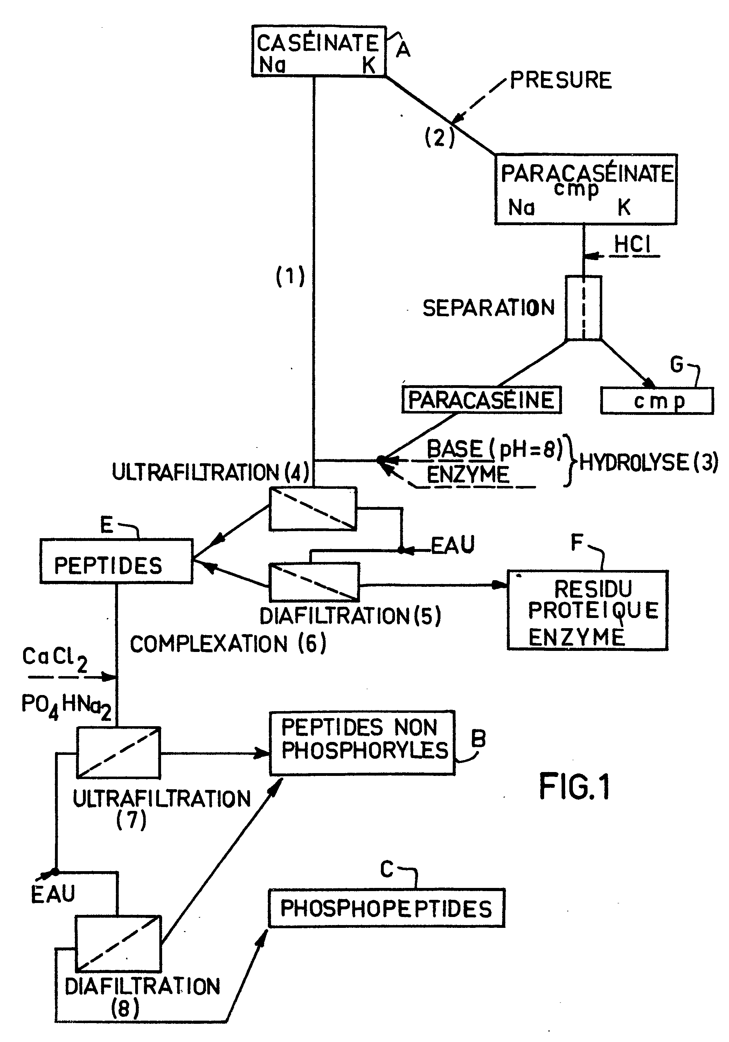

Comme le représente la figure 1, la matière première A à base de caséinate monovalent (sodium ou potassium) peut être directement soumise au procédé (référence 1) ou bien subir un traitement préalable par la voie en variante 2. Selon cette variante, la matière première est hydrolysée par la présure pour fournir une solution contenant le paracaséinate correspondant (sodium ou potassium) et le caséinomacropeptide. La paracaséine est précipitée par acidification (HCl). On effectue ensuite la séparation de la paracaséine précipitée et du caséinomacropeptide. Celui-ci (C.P) constitue un sous-produit G du procédé qui peut être, sans que ces opérations aient été représentées, neutralisé, par exemple par l'hydroxyde de sodium, et ensuite concentré par ultrafiltration après addition de chlorure de calcium.

La matière première du procédé est donc soit la caséinate A, soit son dérivé, la paracaséine.

La première étape du procédé est une hydrolyse (référence 3) en réacteur enzymatique. On a représenté au dessin l'addition d'une base permettant d'atteindre un pH de 8, en vue d'un bon déroulement de l'hydrolyse à l'aide d'une enzyme protéolytique telle que la pancréatine. Le produit de l'hydrolyse est ensuite soumis à une ultrafiltration (4) puis à une diafiltration (5) avec de l'eau. On obtient, d'une part, une fraction peptidique E, et une fraction résiduelle F contenant les protéines et l'enzyme résiduelles. C'est la fraction peptidique E qui est traitée complémentairement dans le procédé. L'étape consécutive est une complexation (référence 6) au cours de laquelle on additionne la fraction E de chlorure de calcium et éventuellement d'un phosphate $PO_4HNa_2$. Après complexation ou agrégation des peptides, le produit est soumis à une ultrafiltration 7, puis à une diafiltration 8 avec de l'eau. On obtient ainsi, finalement, une fraction B (perméat) enrichie en peptides non phosphorylés, et une fraction C enrichie en phosphopeptides.

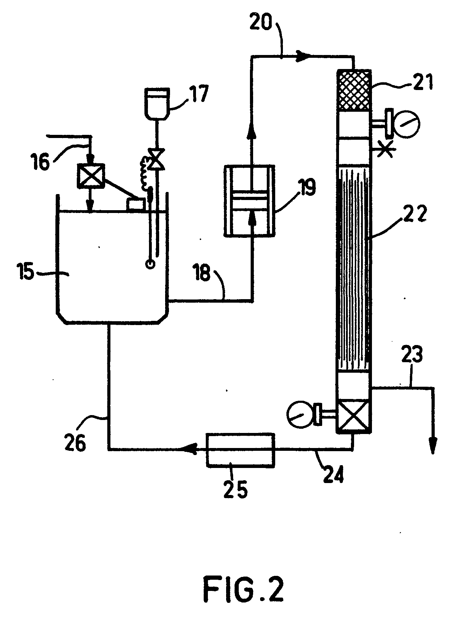

La figure 2 représente un réacteur enzymatique à membrane qui peut être utilisé pour le procédé de l'invention.

Celui-ci comporte d'abord une cuve à réaction désignée par la référence générale 15. L'alimentation continue en phosphocaséinates se fait par la canalisation 16. Un appareil 17 sert à la fois à mesurer et à maintenir le pH dans la cuve de réaction en neutralisant les ions $H^+$ libérés lors de la coupure des liaisons peptidiques. L'appareil était un pH-stat Mettler comprenant un amplificateur de potentiel, un présélecteur du point d'équivalence et une burette automatique de distribution du réactif, ce dernier étant un composé basique tel que mentionné ci-dessus. Aucun encrassement excessif de l'électrode n'a été constaté. Le produit d'hydrolyse prélevé dans la cuve de réaction par la canalisation 18 est acheminé par une pompe pneumatique 19 à membrane. Un exemple pratique est la pompe modèle AMICON LP 20 A, capable de débiter 720 l/h à 1,7 bar. Au refoulement de la pompe, le produit passe dans une canalisation 20 et est amené sur un préfiltre 21 ayant une porosité de 150 microns. La référence 22 désigne le module d'ultrafiltration. Dans un exemple spécifique, on a utilisé le système DC 10 S de la Société AMICON comprenant des cartouches d'ultrafiltration à fibres creuses. Le perméat était récupéré dans une canalisation 23 et constituait l'hydrolysat peptidique recherché. Le rétentat du module 22 était prélevé par la canalisation 24, introduit sur un échangeur 25 et acheminé par la canalisation 26 pour être recyclé dans la cuve de réaction 15.

Les membranes utilisées étaient du type fibres creuses ayant les caractéristiques suivantes :

| Type | Seuil de coupure | Surface | Fabricant |
|------|------------------|---------|-----------|
| H 10 P 5 | 5 000 | 0,9 m² | AMICON |
| H 10 P 10 | 10 000 | | |
| PM 2 | 2 000 | 1,4 m² | ROMICON |
| PM 50 | 50 000 | | |

Exemple 1

Dans cet exemple, on utilise le caséinate de sodium comme matière première.

Les préparations de caséinomacropeptide et des fractions peptidiques selon le procédé décrit dans la figure 1 ont été effectuées en deux étapes :

1) Le caséinate de sodium en solution dans l'eau (3 %) a été hydrolysé en cuve par la présure (20 ml/100 l — présure BOLL 1/10 000) à 37 °C à pH 6,8 pendant 50 minutes. La paracaséine a été ensuite précipitée par acidification à pH 4,6 avec de l'acide chlorhydrique 4 N ; 440 ml d'acide pour 100 l de sérum ont été nécessaires.

Après décantation, le surnageant contenant le CMP a été filtré sur toile, puis centrifugé (1 000 g pendant 8 min) après réajustement du pH à 6,6 par de la potasse 2 N. Cette solution a été ensuite concentrée par ultrafiltration sur membrane après addition de $CaCl_2$ (0,5 g/l). L'appareillage utilisé était un module Amicon type DC 10 équipé de membranes Romicon Hollow Fiber type XM 50 de surface 1,4 m².

Les analyses chimiques des différents produits obtenus au cours de cette fabrication sont rassemblées dans les tableaux II et III.

Tableau III
Composition en acides aminés
(g d'acide aminé pour 100 g)

| | | | | | |
|------|------|------|-----|------|------|
| Asp | 6,9 | Gly | 1,4 | Ile | 10,3 |
| Thr | 9,6 | Ala | 6,1 | Leu | 3,5 |
| Ser | 3,1 | Cyst | ε | Tyr | ε |
| Glu | 23,2 | Val | 8,7 | Phe | 1,7 |
| Pro | 12,7 | Met | 1,5 | Lys | 6,6 |
| | | | | His | 0,6 |
| | | | | Arg | 0,9 |

Tableau II

| | ES | $N_T \times 6,38$ | NPN* | |
|---|-----|-----|-----|-----|
| | | | TCA 6 % | TCA 12 % |
| Caséinate départ (A) | 31,2 | 27,1 | — | — |
| Caséinate après action présure | — | — | 182,5 | 95,4 |
| Lactosérum présure après centrifugation, réajustement du pH et addition de $CaCl_2$ | 3,45 | 1,88 | 166,8 | 105,8 |
| Lactosérum concentré par ultrafiltration ou CPM (G) | 31,3 | 30,1 | 2 649 | 1 191 |
| Perméat fin ultrafiltration | 2,07 | 0,3 % | — | — |

* Composition chimique des produits exprimée en g/kg sauf pour l'azote non protéique (NPN) exprimé en ppm d'azote.

## 0 034 083

2) Le caséinate de sodium en solution dans l'eau à 6,2 % a été hydrolysé dans un réacteur enzymatique à membrane identique à celui représenté sur la figure 2. Les membranes utilisées étaient du type fibres creuses XM 50, d'une surface de 4,9 m². L'enzyme (pancréatine Sigma d'origine bovine d'activité 4 NF) était ajoutée à une concentration de 4 g/litre. Le pH du réacteur était maintenu à 8 par addition d'hydroxyde de potassium 2 N. L'hydrolyse était conduite à 37-40 °C. Le perméat, avant d'être collecté, a été recyclé dans la cuve pendant une heure. Le perméat ainsi obtenu contenait les phosphopeptides et les peptides non phosphorylés. Le perméat a été acidifié à pH 6,4, puis on a provoqué l'agrégation des phosphopeptides par addition de $CaCl_2$ (0,6 %) et de $PO_4HNa_2$ (0,1 %). On a ensuite procédé au fractionnement des deux groupes de peptides en ultrafiltrant et en diafiltrant avec de l'eau afin d'éliminer toute la fraction de peptides non phosphorylés. Les opérations de concentration et diafiltration ont été réalisées sur membranes de type XM 50 à pH 6,5 et à une température de 8 °C. Le concentré diafiltré obtenu correspondait à la fraction phosphopeptidique.

Les analyses des différents produits sont représentées dans les tableaux IV et V suivants :

### Tableau IV
### Composition chimique des produits obtenus

|  |  | ES g/kg | $N_T \times 6{,}38$ g/kg | Ca g/kg | Cendres g/kg |
|---|---|---|---|---|---|
| (A) | Caséinate départ | 62,3 | 54,2 | — | — |
| (E) | Peptides totaux | 62,3 | 45,9 | — | — |
| (B) | Peptides non phosphorylés | 53,7 | 47,3 | — | — |
| (C) | Phosphopeptides | 15,0 | 7,90 | 2,35 | 6,3 |

### Tableau V
### Compositions en acides aminés des phosphopeptides
### (g d'acide aminé pour 100 g)

| Asp | 5,6 | Cys | ε |
|---|---|---|---|
| Thr | 3,3 | Met | 0,45 |
| Ser | 18,5 | Ile | 9,5 |
| Pro | 4,7 | Leu | 2,9 |
| Glu | 36,8 | Tyr | ε |
| Gly | 1,7 | Phe | ε |
| Ala | 2,8 | Lys | 0,9 |
| Val | 8,7 | His | 0,5 |
|  |  | Arg | 0,3 |

Des exemples illustratifs concernant l'application des produits de l'invention seront indiqués ci-après, à titre nullement limitatif.

### Exemple 2

Cet exemple concerne un produit de réanimation destiné à un usage : par voie entérale chez des malades nécessitant un apport protéique de l'ordre de 7 à 15 % de l'apport calorique total (ACT), par exemple dans les affections suivantes : mucoviscidose ou fibrose kystique du pancréas, insuffisance rénale, malades présentant une atteinte infectieuse ou inflammatoire de la paroi intestinale, détresse nutritionnelle exigeant un anabolisme intense, une charge rénale osmotique et une charge en ions $H^+$ réduites. Ces protéines sont amenées de préférence sous forme pré-digérée.

Exemple de formule centésimale selon l'invention :

| | | |
|---|---|---|
| Phosphopeptides | de 4 à 8 % | ⎫ |
| Peptides de lactosérum | 60 % | ⎬ 2,50 g |
| Peptides de caséine | de 32 à 36 % | |
| CMP | de 0 à 4 % | ⎭ |

— lipides :
mélange à parties égales de :

| | | |
|---|---|---|
| huile de beurre | 0,5 g | ⎫ |
| triglycéride à chaîne moyenne (TCM) | 0,5 g | |
| huile de maïs | 0,5 g | ⎬ 4,10 g |
| huile de tournesol | 0,5 g | |
| monostéarate de glycérol | 2,1 g | ⎭ |

(Fortsetzung)

— glucides :
  polymères de glucose        10   g       }
  glucose                 1,5 g      }    13,00 g
  galactose             1,5 g      )

— vitamines :

  A, D, E, $B_1$, $B_2$, PP, $B_5$, $B_6$, $B_{12}$, acide folique, Biotine, Vit. C     } selon les recommandations FAO/OMS

— éléments minéraux :
  (calcium, sodium, potassium, magnésium, phosphore, zinc, fer, cuivre, manganèse, chlore, iode)     } 0,455 g

— eau distillée                                               q.s.p.   100

## Exemple 3

Cet exemple concerne un produit de réanimation destiné à un usage par voie entérale chez des malades nécessitant un apport protéique de l'ordre de 12 à 25 % de l'Apport Calorique Total, sous forme de protéines. Une telle alimentation est appropriée à toute situation nécessitant un anabolisme azoté important et une surcharge en p⁻ organique.

Exemple de formule centésimale

— mélange de petits peptides selon l'invention :
  phosphopeptides            de  8 à 28 %    }
  peptides de lactosérum      de 22 à 60 %   } de 3 à 6,25 g
  peptides de caséine         de 12 à 70 %   )

— lipides :
  T.C.M.                        2,30 g    }
  huile très riche en acides gras essentiels    0,50 g    } 2,90 g
  émulsifiant                      0,10 g    )

— glucides :
  petits polymères de glucose       de 8,25 à 10,5 g   }
  glucose                   de 2   à   2,5 g   } de 12,25 à 15,5 g
  galactose                 de 2   à   2,5 g   )

— vitamines :
  A, D, E, $B_1$, $B_2$, PP, $B_5$, $B_6$, $B_{12}$, acide folique, Biotine, Vitamine C,     } selon les recommandations FAO/OMS

— éléments minéraux :
  (calcium, sodium, potassium, magnésium, phosphore, zinc, fer, cuivre, manganèse, chlore, iode)     } 0,455 g

— eau distillée                                               q.s.p.   100

## Exemple 4

Cet exemple concerne un produit de réanimation à un usage par voie entérale chez des malades nécessitant un apport protéique de l'ordre de 7 à 12 % de l'A.C.T., notamment dans les affections suivantes : mucoviscidose ou fibrose kystique du pancréas, insuffisance rénale, malades présentant une atteinte infectieuse ou inflammatoire de la paroi intestinale, détresse nutritionnelle exigeant un anabolisme intense et une surcharge rénale osmotique, une surcharge en ions H⁺ réduites. Ces protéines sont amenées de préférence sous forme pré-digérée.

Exemple de formule centésimale :

— mélange de petits peptides selon l'invention :
  phosphopeptides             4 %        }
  peptides de caséine         36 %      }
  peptides de lactosérum      40 %      } 2,50 g
  CMP                        20 %      )

— lipides :
mélange à parties égales de :

| | | |
|---|---|---|
| huile de beurre | 0,5 g | |
| TCM | 0,5 g | |
| huile de maïs | 0,5 g | 4,10 g |
| huile de tournesol | 0,5 g | |
| monostéarate de glycérol | 2,1 g | |

— glucides :

| | | |
|---|---|---|
| polymères de glucose | 10 g | |
| glucose | 1,5 g | 13,00 g |
| galactose | 1,5 g | |

— vitamines :

A, D, E, B$_1$, B$_2$, PP, B$_5$, B$_6$, B$_{12}$, acide folique, Biotine, Vit. C     selon les recommandations FAO/OMS

— éléments minéraux :
(calcium, sodium, potassium, magnésium, phosphore, zinc, fer, cuivre, manganèse, chlore, iode)     0,455 g
— eau distillée     q.s.p. 100

Le mélange de peptides contenant les phosphopeptides de l'invention peut avantageusement comprendre les peptides obtenus conformément au procédé décrit dans la demande de brevet EP-A-0022019. On rappellera qu'un tel hydrolysat peptidique ne contient pratiquement pas de protéines résiduelles et que 50 % des peptides contiennent 2 à 5 acides aminés. Plus particulièrement, l'hydrolysat contient 70 à 90 % de l'azote présent sous forme de peptides ayant un nombre d'acides aminés inférieur à 10. Sous une forme spécifique, l'hydrolysat répond à l'aminogramme ci-après :

Aminogramme :

| | | | | |
|---|---|---|---|---|
| Ile | 6,0 | Arg | 2,7 | |
| Leu | 9,9 | His | 1,7 | |
| Lys | 9,2 | Ala | 4,9 | |
| Cys | 1,8 | Asp | 9,5 | |
| Phe | 3,2 | Glu | 17,6 | |
| Thr | 6,7 | Gly | 1,7 | |
| Tyr | 3,6 | Pro | 6,2 | |
| Trp | 2,0 | Ser | 5,5 | |
| Val | 5,5 | Met | 2,0 | |

Le procédé pour l'obtention d'un tel hydrolysat consiste à effectuer d'abord l'ultrafiltration du lactosérum puis son hydrolyse enzymatique, et il est plus particulièrement remarquable en ce qu'on met en contact le rétentat d'ultrafiltration avec une enzyme protéolytique capable de reconstituer la digestion protéique qui prend place in vivo dans l'organisme humain, ladite enzyme étant de préférence la pancréatine, l'hydrolyse étant conduite jusqu'à ce que le produit ne contienne plus de protéines résiduelles, c'est-à-dire ne présente aucun azote précipitable par l'acide trichloroacétique à 12 %, après quoi on récupère l'hydrolysat obtenu qui représente l'hydrolysat enzymatique total recherché.

**Revendications**

1. Procédé de traitement des phosphocaséinates de cations monovalents, tels que les phosphocaséinates de sodium, de potassium ou d'ammonium, ou des dérivés desdits phosphocaséinates, tels que la paracaséine, caractérisé en ce qu'on les soumet à une hydrolyse enzymatique en soi connue à l'aide d'au moins une enzyme protéolytique capable de reconstituer la digestion protéique qui prend place in vivo dans l'organisme humain, ladite hydrolyse enzymatique étant réalisée à un pH entre 7 et 9 et à une température de 37 à 45 °C, en ce qu'on soumet l'hydrolysat obtenu à au moins une ultrafiltration sur des membranes laissant passer dans le perméat tous les peptides de l'hydrolysat, en ce qu'on ajoute au perméat au moins un sel de cation bivalent pouvant former des agrégats avec la fraction phosphorylée desdits peptides, ce qui conduit à une solution contenant essentiellement des agrégats de phosphopeptides et des peptides non phosphorylés, et en ce qu'on sépare par au moins une ultrafiltration les peptides non phosphorylés et les phosphopeptides, lesquels ont une grosseur de particule supérieure, par mise en contact de la solution avec au moins une membrane capable de retenir les phosphopeptides.

2. Procédé selon la revendication 1, caractérisé en ce qu'on traite au préalable les phosphocaséinates de cations monovalents en les additionnant de présure, en précipitant la paracaséine contenue dans le produit d'hydrolyse, notamment par acidification à pH 4,6, et en séparant la paracaséine précipitée de la solution surnageante qui contient du caséinomacropeptide CMP.

3. Procédé selon l'une des revendications 1 ou 2, caractérisé en ce qu'on effectue l'hydrolyse enzymatique dans un dispositif qui combine un équipement d'ultrafiltration à un réacteur enzymatique, constituant ainsi un réacteur enzymatique à membrane.

4. Procédé selon la revendication 3, caractérisé en ce qu'on réalise en continu l'hydrolyse enzymatique en introduisant la matière première à base de caséine dans une zone de réaction en vue d'une mise en contact intime avec l'enzyme, on soutire en continu le produit de réaction en l'amenant de la zone de réaction dans une zone d'ultrafiltration, de laquelle on soutire également en continu un perméat qui constitue l'hydrolysat peptidique.

5. Procédé selon l'une quelconque des revendications 1 à 4, caractérisé en ce qu'on ajuste le pH entre 7 et 8,5, par exemple à 8 environ, au cours de l'hydrolyse enzymatique.

6. Procédé selon l'une quelconque des revendications 1 à 5, caractérisé en ce qu'à titre d'enzyme, on utilise une pancréatine sous forme d'extrait pancréatique naturel, ou des mélanges synthétiques de trypsine et d'alphachymotrypsine.

7. Procédé selon l'une quelconque des revendications 1 à 6, caractérisé en ce que la température d'hydrolyse est choisie entre 37 et 40 °C et avantageusement, de l'ordre de 37 °C.

8. Procédé selon l'une quelconque des revendications 1 à 7, caractérisé en ce que, dans le réacteur enzymatique à membrane, on utilise des membranes capables de retenir efficacement l'enzyme, dont le seuil de coupure se situe de préférence entre 2 000 et 50 000, et avantageusement entre 2 000 et 10 000.

9. Procédé selon l'une quelconque des revendications 1 à 8, caractérisé en ce qu'on remplace au moins partiellement les cations bivalents des sels de phosphopeptides, par des oligoéléments tels que le fer, le zinc, le cuivre, le chrome, le nickel, le cobalt, le manganèse ou le sélénium, en utilisant, lors de la purification des phosphopeptides une solution contenant le ou les oligoéléments à introduire, ce qui conduit à des sels organophosphorés avec des oligoéléments liés.

10. Produits tels qu'obtenus par le procédé selon l'une quelconque des revendications 1 à 8, consistant en des peptides non phosphorylés.

11. Produits tels qu'obtenus par le procédé selon l'une quelconque des revendications 1 à 8, consistant en des sels organophosphorés formés de phosphopeptides et de cations bivalents, tels que le calcium et/ou le magnésium.

12. Sels de phosphopeptides et d'oligoéléments, tels qu'obtenus par le procédé de la revendication 9, en particulier de fer, zinc, cuivre, chrome, nickel, cobalt, manganèse et sélénium.

13. Sels organophosphorés de phosphopeptides et d'éléments minéraux, caractérisés par les paramètres ci-après :
somme des acides aminés aromatiques $< 4\,\%$
taux de sérine supérieur à $8\,\%$ et inférieur à $20\,\%$
rapport $Ca + Mg + P/N_T > 0,2$, où $N_T$ représente l'azote total $\times\ 6,38$
teneur en acides aminés libres $< 3\,\%$.

14. Application des produits selon l'une quelconque des revendications 11, 12 et 13, comme aliments ou compléments alimentaires répondant à des besoins spécifiques nutritionnels.

15. Aliments diétiques ou de nutrition thérapeutique contenant un produit selon l'une quelconque des revendications 11, 12 et 13, en association éventuelle avec un véhicule neutre convenant à l'alimentation orale ou entérale.

16. Aliments selon la revendication 15, contenant en outre des sources complémentaires d'acides aminés essentiels.

17. Application des produits selon l'une quelconque des revendications 11, 12, 13, à titre de médicaments.

18. Composition pharmaceutique contenant une quantité efficace d'au moins un produit selon l'une quelconque des revendications 11, 12 et 13, en association avec un véhicule pharmaceutiquement acceptable, convenant notamment à l'administration entérale.

19. Composition selon la revendication 18, contenant au moins un sel organophosphoré de phosphopeptide selon la revendication 11 ou 13, constituant un supplément minéraloprotidique riche en phosphore organique, en calcium et/ou magnésium.

20. Composition selon la revendication 18, contenant au moins un sel de phosphopeptide selon la revendication 12, pour remédier aux malabsorptions digestives induisant des carences en oligoéléments.

**Claims**

1. Method for treating phosphocaseinates of monovalent cations, such as sodium, potassium or ammonium phosphocaseinates, or derivatives of said phosphocaseinates, such as paracasein, characterized in that they are subjected to an enzymatic hydrolysis known per se by means of at least one proteolytic enzyme capable of reproducing the proteic digestion which occurs in vivo in the human body, said enzymatic hydrolysis being effected at a pH from 7 to 9 and at a temperature of 37 to 45 °C, in that the hydrolyzate obtained is subjected to at least one ultrafiltration on membranes which allow all the peptides of the hydrolyzate to pass into the permeate, in that the permeate is added with at least one bivalent cation salt capable of forming aggregates with the phosphorylated fraction of said peptides, this leading to a solution which essentially contains aggregates of phosphopeptides and non phosphorylated peptides, and in that separation is effected by at least one ultrafiltration between the non phosphorylated peptides and the phosphopeptides, these having a larger particle size, by bringing the solution into contact with at least one membrane capable of retaining the phosphopeptides.

2. Method according to claim 1, characterized in that the phosphocaseinates of monovalent cations are previously treated by adding rennet thereto, by precipitating the paracasein contained in the hydrolysis product, especially by acidification to pH 4.6, and by separating the precipitated paracasein from the supernatant solution which contains caseinomacropeptide CMP.

3. Method according to either of claims 1 or 2, characterized in that the enzymatic hydrolysis is effected in a device which combines an ultrafiltration equipment with an enzymatic reactor, thus constituting a membrane enzymatic reactor.

4. Method according to claim 3, characterized in that the enzymatic hydrolysis is performed continuously by introducing the casein-based raw material into a reaction zone for intimate contacting with the enzyme, the reaction product is withdrawn continuously by being moved from the reaction zone into an ultrafiltration zone, wherefrom there is withdrawn also continuously a permeate which constitues the peptidic hydrolyzate.

5. Method according to any of claims 1 to 4, characterized in that the pH is adjusted between 7 and 8.5, for example to about 8, during the enzymatic hydrolysis.

6. Method according to any of claims 1 to 5, characterized in that there is used as an enzyme, a pancreatine under the form of natural pancreatic extract, or synthetic mixtures of trypsine and alphachymotrypsine.

7. Method according to any of claims 1 to 6, characterized in that the hydrolysis temperature is selected between 37 and 40 °C and advantageously, of the order of 37 °C.

8. Method according to any of claims 1 to 7, characterized in that, in the membrane enzymatic reactor, there are used membranes capable of effectively retaining the enzyme, which have a cut off threshold ranging preferably from 2 000 to 50 000, and advantageously from 2 000 to 10 000.

9. Method according to any of claims 1 to 8, characterized in that the bivalent cations of the phosphopeptide salts are at least partly replaced by oligoelements such as iron, zinc, copper, chromium, nickel, cobalt, manganese or selenium, by using, when purifying the phosphopeptides a solution containing the oligoelement or oligoelements to be introduced, which leads to organophosphorated salts with bonded oligoelements.

10. Products such as obtained by the method according to any of claims 1 to 8, consisting in non phosphorylated peptides.

11. Products such as obtained by the method according to any of claims 1 to 8, consisting of organophosphorated salts formed of phosphopeptides and of bivalent cations, such as calcium and/or magnesium.

12. Salts of phosphopeptides and oligoelements, such as obtained by the method of claim 9, especially of iron, zinc, copper, chromium, nickel, cobalt, manganese and selenium.

13. Organophosphorated salts of phosphopeptides and mineral elements, characterized by the following parameters :
sum of the aromatic amino-acids < 4 %
amount of serine higher than 8 % and lower than 20 %
$Ca + Mg + P/N_T$ ratio > 0.2, where $N_T$ represents the total nitrogen $\times$ 6.38
free amino-acid content < 3 %.

14. Application of the products according to any of claims 11, 12 and 13, as aliments or food complements meeting specific nutritional requirements.

15. Dietetic or therapeutic nutrition aliments containing a product according to any of claims 11, 12 and 13, in possible association with a neutral carrier suitable for oral or enteral nutrition.

16. Aliments according to claim 15, further containing complementary sources of essential aminoacids.

17. Application of products according to any of claims 11, 13, 14, as medicaments.

18. Pharmaceutical composition containing an effective amount of at least one product according to any of claims 11, 12 and 13, in association with an acceptable pharmaceutical carrier, suitable more particularly for enteral administration.

19. Composition according to claim 18, containing at least one organophosphorated salt of phosphopeptide according to claim 11 or 13, constituting a mineraloprotidic supplement rich in organic phosphorus, and in calcium and/or magnesium.

20. Composition according to claim 18, containing at least one phosphopeptide salt according to claim 12, to remedy digestive misabsorptions inducing deficiencies of oligoelements.

**Ansprüche**

1. Verfahren zur Behandlung von Phosphokaseinaten von einwertigen Kationen wie beispielsweise

14

Phosphokaseinaten von Natrium, Kalium oder Ammonium oder von Derivaten der genannten Phosphokaseinate, z. B. Parakasein, dadurch gekennzeichnet, daß man sie einer an sich bekannten enzymatischen Hydrolyse mit Hilfe wenigstens eines proteolytischen Enzyms unterwirft, das die in vivo im menschlichen Organismus stattfindende Eiweißverdauung zu rekonstituieren vermag, wobei die enzymatische Hydrolyse bei einem pH-Wert zwischen 7 und 9 und bei einer Temperatur von 37 bis 45 °C durchgeführt wird, daß man das erhaltene Hydrolysat wenigstens einer Ultrafiltration auf Membranen unterwirft, die im Permeat alle Peptide des Hydrolysats durchlassen, daß man dem Permeat wenigstens ein Salz eines zweiwertigen Kations zusetzt, das mit der phosphorylierten Fraktion der genannten Peptide Aggregate zu bilden vermag, was zu einer Lösung führt, die im wesentlichen Aggregate von Phosphopeptiden und nicht phosphorylierte Peptide enthält, und daß man durch wenigstens eine Ultrafiltration die nicht phosphorylierten Peptide und die Phosphopeptide abtrennt, die eine grobere Teilchengröße haben, indem man die Lösung mit wenigstens einer Membran, die die Phosphopeptide zurückzuhalten vermag, in Berührung bringt.

2. Verfahren nach Anspruch 1, dadurch gekennzeichnet, daß man die Phosphokaseinate von einwertigen Kationen einer Vorbehandlung unterwirft, indem man sie mit Lab versetzt, das im Hydrolysenprodukt enthaltene Parakasein insbesondere durch Ansäuerung auf pH 4,6 ausfällt und das ausgefällte Parakasein von der obenstehenden Lösung, die Kaseinomakropeptid CMP enthält, abtrennt.

3. Verfahren nach einem der Ansprüche 1 oder 2, dadurch gekennzeichnet, daß man die enzymatische Hydrolyse in einer Vorrichtung durchführt, die eine Ultrafiltrationsvorrichtung mit einem enzymatischen Reaktor kombiniert und in dieser Weise einen enzymatischen Reaktor mit Membran bildet.

4. Verfahren nach Anspruch 3, dadurch gekennzeichnet, daß man die enzymatische Hydrolyse Kontinuierlich durchführt, indem man das Ausgangsmaterial auf Basis von Kasein in eine Reaktionszone mit dem Ziel einer innigen Inberührungbringung mit dem Enzym einführt, das Reaktionsprodukt kontinuierlich abzieht, indem man es aus der Reaktionszone in eine Ultrafiltrationszone führt, aus der man ebenfalls kontinuierlich ein Permeat abzieht, das das Peptidhydrolysat darstellt.

5. Verfahren nach einem beliebigen der Ansprüche 1 bis 4, dadurch gekennzeichnet, daß man den pH-Wert im Verlauf der enzymatischen Hydrolyse zwischen 7 und 8,5, beispielsweise auf etwa 8 einstellt.

6. Verfahren nach einem beliebigen der Ansprüche 1 bis 5, dadurch gekennzeichnet, daß man als Enzym ein Pankreatin in Form von natürlichem Pankreasextrakt oder synthetische Gemische von Trypsin und α-Chymotrypsin verwendet.

7. Verfahren nach einem beliebigen der Ansprüche 1 bis 6, dadurch gekennzeichnet, daß man die Temperatur der Hydrolyse zwischen 37 und 40 °C und vorteilhaft in der Größenordnung von 37 °C wählt.

8. Verfahren nach einem beliebigen der Ansprüche 1 bis 7, dadurch gekennzeichnet, daß man im enzymatischen Membranreaktor Membranen verwendet, die Wirksam das Enzym, dessen Schnittschwelle vorzugsweise zwischen 2 000 und 50 000 und vorteilhaft zwischen 2 000 und 10 000 liegt, zurückzuhalten vermögen.

9. Verfahren nach einem beliebigen der Ansprüche 1 bis 8, dadurch gekennzeichnet, daß man die zweiwertigen Kationen der Phosphopeptidsalze wenigstens teilweise durch Oligoelemente, z. B. Eisen, Zink, Kupfer, Chrom, Nickel, Kobalt, Mangan oder Selen, ersetzt, indem man bei der Reinigung der Phosphopeptide eine Lösung verwendet, die das oder die einzuführenden Oligoelemente enthält, was zu Organophosphorsalzen mit den gebundenen Oligoelementen führt.

10. Produkte, wie sie nach dem Verfahren gemäß einem beliebigen der Ansprüche 1 bis 8 erhalten werden und aus nicht phosphorylierten Peptiden bestehen.

11. Produkte, wie sie nach dem Verfahren gemäß einem beliebigen der Ansprüche 1 bis 8 erhalten werden und aus Organophosphorsalzen bestehen, die aus Phosphopeptiden und zweiwertigen Kationen, z. B. Calcium und/oder Magnesium, gebildet werden.

12. Salze von Phosphopeptiden und Oligoelementen, wie sie nach dem Verfahren des Anspruchs 9 erhalten werden, insbesondere von Eisen, Zink, Kupfer, Chrom, Nickel, Kobalt, Mangan und Selen.

13. Organophosphorsalze von Phosphopeptiden und mineralischen Elementen, gekennzeichnet durch die nachstehenden Parameter :
Summe der aromatischen Aminosäuren < 4 %
Seringehalt über 8 % und unter 20 %
Verhältnis Ca + Mg + $P/N_T$ > 0,2, worin $N_T$ Gesamtstickstoff × 6,38 darstellt
Gehalt an freien Aminosäuren < 3 %.

14. Verwendung der Produkte gemäß einem beliebigen der Ansprüche 11, 12 und 13 als Nahrungsmittel oder Nahrungsmittelergänzungen für speziellen Nährstoffbedürfnissen.

15. Diätetische Nahrungsmittel oder Nahrungsmittel für die therapeutische Ernährung, enthaltend ein Produkt gemäß einem beliebigen der Ansprüche 11, 12 und 13 gegebenenfalls in Verbindung mit einem für die orale oder enterale Ernährung geeigneten neutralen Träger.

16. Nahrungsmittel nach Anspruch 15, enthaltend außerdem ergänzende Quellen von essentiellen Aminosäuren.

17. Anwendung der produkte nach einem beliebigen der Ansprüche 11, 12, 13 als Medikamente.

18. Arzneimittelzubereitung, enthaltend eine wirksame Menge wenigstens eines Produkts gemäß einem beliebigen der Ansprüche 11, 12 und 13 in Verbindung mit einem pharmazeutisch unbedenklichen Träger, der sich insbesondere für die enterale Verabreichung eignet.

19. Arzneimittelzubereitung nach Anspruch 18, enthaltend wenigstens ein Organophosphorsalz eines Phosphopeptids gemäß Anspruch 11 oder 13 und darstellend eine an organischem Phosphor, an Calcium und/oder Magnesium reiche Mineraloeiweißergänzung.

20. Arzneimittelzubereitung nach Anspruch 18, die wenigstens ein Phosphopeptidsalz gemäß Anspruch 12 enthält, zur Abhilfe von digestiven und intestinalen Resorptionsstörungen, die das Fehlen oder Mängel von Oligoelementen auslösen.

FIG.1

FIG.2